Europäisches Patentamt

European Patent Office

Office européen des brevets

(11) Numéro de publication: **0 223 176 B1**

# (12) FASCICULE DE BREVET EUROPEEN

(45) Date de publication de fascicule du brevet:
**22.05.91**

(51) Int. Cl.⁵: **A61M 25/10**

(21) Numéro de dépôt: **86115561.2**

(22) Date de dépôt: **10.11.86**

(54) **Cathéter à commande à distance à micro-ballonnet.**

(30) Priorité: **21.11.85 CH 4961/85**

(43) Date de publication de la demande:
**27.05.87 Bulletin 87/22**

(45) Mention de la délivrance du brevet:
**22.05.91 Bulletin 91/21**

(84) Etats contractants désignés:
**AT BE CH DE ES FR GB GR IT LI LU NL SE**

(56) Documents cités:
**EP-A- 0 161 045**      **DE-B- 2 954 391**
**FR-A- 372 096**        **FR-A- 1 278 965**
**GB-A- 2 054 385**      **GB-A- 2 127 294**
**US-A- 4 150 676**      **US-A- 4 215 703**
**US-A- 4 456 017**

(73) Titulaire: **SARCEM SA**
**27 rue Cardinal-Journet Case Postale 371**
**CH-1217 Meyrin 1(CH)**

(72) Inventeur: **Jeanmonod, Maurice**
**47, avenue de Vaudagne**
**CH-1217 Meyrin(CH)**
Inventeur: **Bonello, Philippe**
**26, chemin du Pommier**
**CH-1218 Grand-Saconnex(CH)**

(74) Mandataire: **Micheli, Michel-Pierre et al**
**MICHELI & CIE Rue de Genève 122 Case**
**postale 61**
**CH-1226 Genève-Thônex(CH)**

Rank Xerox (UK) Business Services

**Description**

La présente invention a pour objet un cathéter à commande à distance à micro-ballonnet dont les applications principales pourraient se situer en technique de soins dans le cadre des maladies cardio-vasculaires au niveau des vaisseaux coronaires.

Si le traitement du rétrécissement du calibre artériel dit traitement de la sténose se fait couramment par procédé de dilatation au moyen d'un instrument nommé cathéter à ballonnet gonflable, il n'est pas toujours simple au travers des multiples ramifications circulatoires de placer ledit ballonnet à l'intérieur de la sténose, opération naturellement d'autant plus difficile que le rétrécissement est important. Pour certaines sténoses en particulier et par le fait que le canal artériel est réduit à des dimensions quasi inexistantes, il n'est plus possible d'utiliser le cathéter traditionnel jugé de diamètre beaucoup trop important.

On a alors proposé des guides pour cathéter tels que par exemple décrit dans le document US-A-4.456.017 comprenant un tube muni à son extrémité distale d'un doigt évidé et flexible maintenu à l'état de repos coaxialement au tube par son élasticité propre. Cette tête est constituée par un ressort à boudin ayant des spires disjointes. Le cathéter-guide comporte encore un organe flexible de traction fixé à l'extrémité de la tête en un point excentré et s'étendant au travers du tube jusqu'à un organe de commande. Un tel dispositif peut être très fin et de par son orientabilité être introduit dans de petit vaisseau sanguin. Une fois en place il sert de support ou de guide à un cathéter à ballonnet qui est mis en place par coulissement sur ce guide. L'inconvénient de ce dispositif réside dans la nécessité de procéder à deux interventions, la mise en place du guide puis à celle du cathéter avant de pouvoir procéder à la dilatation voulue.

On connaît également du document EP-A-0 161 045 un cathéter double comprenant une extrémité de faible diamètre munie d'une partie de plus grand diamètre permettant l'extraction de liquide de cavités humaines. On connaît enfin du document FR-A-1 278 965 un cathéter muni d un dispositif de propulsion formé de ballonnets expansibles servant à fixer alternativement certaines parties du cathéter dans un vaisseau pendant l'avancement d une autre partie de ce cathéter.

La présente invention a pour objet un cathéter pour le traitement de la sténose mais d'un très petit diamètre. Ce cathéter se distingue par les caractéristiques énumérées à la revendication 1.

La figure 1 représente une coupe dans son principe du cathéter, la gaine tubulaire et le ballonnet étant deux éléments bien distincts l'un de l'autre.

La figure 2 représente le cathéter dont la gaine tubulaire et le ballonnet ne forment qu'une seule et même pièce.

La figure 3 représente le cathéter dans l'une ou l'autre des deux versions ci-dessus, mais dont l'extrémité de la partie formant le ballonnet ouverte d'origine est fixée de manière étanche sur la bouterolle.

Le cathéter à commande à distance à micro-ballonnet représenté au dessin comprend un tube 1 à l'extrémité duquel est monté coaxialement la tête du cathéter composée d'un ressort boudin cylindrique 2 dont la base est emmanchée sur un embout percé 3 lui-même chassé en partie à l'intérieur du tube 1, d'une bouterolle 4 dont la tige est emmanchée sur l'autre extrémité du ressort boudin cylindrique 2, et d'un élément flexible de traction 5 dont le point de fixation à la bouterolle 4 est décentré par rapport à l'axe de cette dernière. Par ailleurs, nous trouvons un élément-bâti 6 fixé étanche sur le tube 1, une pièce en partie cylindrique 7 à laquelle est fixé l'élément flexible de traction 5, un o-ring 8 assurant l'étanchéité entre l'élément-bâti 6 et la pièce cylindrique 7, une gaine tubulaire 9, un ballonnet gonflable 10.

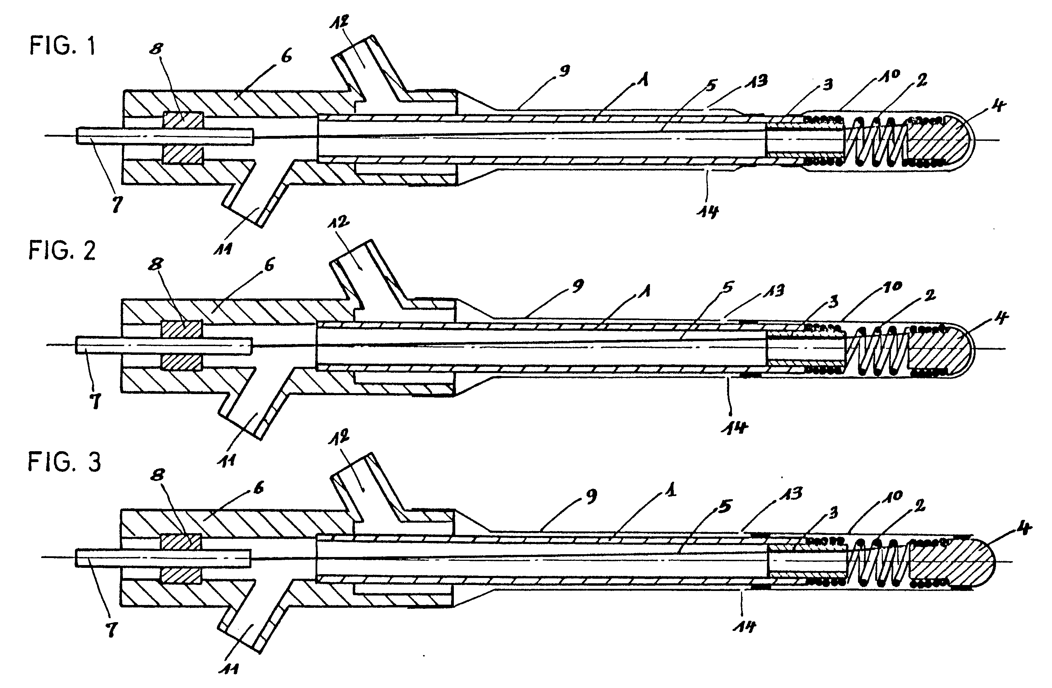

Pour diriger l'extrémité nommée tête du cathéter coiffée de son ballonnet gonflable 10 tel qu'il se présente à la figure 1 dans l'origine de l'une ou l'autre des ramifications circulatoires puis à l'intérieur des zones à traiter, l'utilisateur dispose pour ce faire de trois degrés de liberté, à savoir avance ou recul de l'ensemble et par conséquent de la tête, rotation de l'ensemble sur lui-même dans un sens ou dans l'autre par rapport à son axe, inclinaison de la tête ou redressement de cette dernière, la mise en oeuvre de ces deux dernières fonctions étant possible grâce à la force en arrière allant à l'encontre de l'action élastique déployée par le ressort boudin cylindrique 2 ou en avant que l'utilisateur imprimera sur la pièce en partie cylindrique 7 et par conséquent sur l'organe flexible de traction 5 gainé par le tube 1, la pièce en partie cylindrique 7 étant saisissable directement et manuellement par son extrémité arrière ou attachée par cette même extrémité à un mécanisme classique non représenté sur le dessin du type micrométrique par exemple.

En ce qui concerne le gonflage du ballonnet 10, le fluide sous pression est introduit par le canal 11 de l'élément-bâti 6 pour arriver au ballonnet 10 au travers du tube 1, la base du ballonnet 10 étant fixée sur le tube 1 de manière étanche.

Reste la fonction classique et bien connue consistant à introduire du liquide de contraste à l'intérieur de la ramification circulatoire considérée et ceci à des fins de repère pour lecture en examen radioscopique. Pour cela, le liquide de contraste est injecté par le canal 12 de l'élément-bàti 6 envahissant l'espace situé entre le tube 1 et la gaine tubulaire 9 pour sortir à l'extérieur par les deux orifices 13 et 14 pratiqués dans cette dernière, cette opération étant possible grâce au fait que l'une des extrémités de la gaine tubulaire 9 est fixée étanche sur l'élément-bàti 6, alors que l'autre extrémité est fixée également étanche mais sur le tube 1.

**Revendications**

1. Cathéter à commande à distance à micro-ballonet pour le traitement des sténoses comprenant un tube (1) dont l'une des extrémités est fixée à la partie inférieure de la tête du cathéter, une gaine tubulaire (9) entourant le tube (1), dont l'une des extrémités est fixée de manière étanche sur l'extrémité du tube (1) qui porte la tête cette gaine constituant avec la paroi extérieure du tube (1) un conduit pour le passage d'un fluide, cette tête étant composée d'un ressort boudin (2) formé d'une ou de plusieurs spires disjointives, d'une pièce d'extrémité (4) fixée à l'extrémité supérieure du ressort (2) et étant entourée d'une enveloppe (10) fixée sur l'extrémité du tube (1) portant la tête de manière étanche constituant le ballonet gonflable, cette tête ayant la forme d'un doigt évidé et flexible maintenu à l'état repos pratiquement droit et dans une position approximativement coaxiale au tube (1) par l'action élastique du ressort (2); un organe flexible de traction (5) s'étendant de la pièce d'extrémité (4) de la tête du cathéter à l'arrière du tube (1) en passant à l'intérieur de ce dernier, cet organe flexible de traction (5) étant décentré par rapport à l'axe de ce ressort (2), l'autre extrémité de cet organe flexible (5) étant fixée à un dispositif de commande 7.

2. Cathéter à commande à distance selon la revendication 1, caractérisé par le fait qu'il comporte un élément-bâti (6) comportant deux canaux latéraux (11,12) permettant, l'un (12) la communication avec l'espace situé entre l'extérieur du tube (1) et l'intérieur de la gaine tubulaire (9), cette gaine tubulaire (9) étant montée de manière étanche par l'autre de ses extrémités sur l'élément-bâti (6), cette gaine tubulaire (9) étant percée latéralement (13,14) de manière à permettre au liquide injecté par ce canal (12) de sortir à l'extérieur du cathéter; l'autre canal (11) permettant, par l'intérieur du tube (1), le gonflement du ballonnet.

3. Cathéter à commande à distance selon la revendication 1 ou la revendication 2, caractérisé par le fait que la gaine (9) et l'enveloppe (10) sont réalisées en une seule pièce, le lien commun délimitant la gaine tubulaire (9) de l'enveloppe (10) étant fixé sur le tube (1) de manière étanche.

4. Cathéter à commande à distance selon la revendication 1, 2 ou 3, caractérisé par le fait que le tube (1) est fin et flexible.

5. Cathéter à commande à distance selon la revendication 1, caractérisé par le fait que l'extrémité du tube (1) est façonnée en forme de téton dont le diamètre extérieur est plus petit que le diamètre extérieur du tube (1), le ressort boudin (2) étant emmanché par sa base sur cette extrémité en forme de téton; et par le fait que la partie supérieure du ressort boudin (2) est emmanchée sur la tige de la pièce (4) à laquelle est fixée l'une des extrémités de l'organe flexible de traction (5).

6. Cathéter à commande à distance selon la revendication 5, caractérisé par le fait que l'une des extrémités de l'organe flexible de traction (5) est fixée à la tête du cathéter par pincement entre le ressort à boudin (2) et la pièce (4).

**Claims**

1. Remote controlled catheter having a micro-baloon for the treatment of stenoses comprising a tube (1) one end of which is fastened to the lower portion of the head of the catheter, a tubular covering (9) covering the tube (1), one end of which is fixed tightly on the end of the tube (1) carrying the head, this covering forming with the outside wall of the tube (1) a duct for the passage of a fluid, this head comprising a coil spring (2) formed of one or several disjointed turns, an end piece (4) fixed to the upper end of the spring (2) and surrounded by an envelope (10) fixed to the end of the tube (1) carrying the head in a tight manner forming the inflatable baloon, this head having the shape of an hollow finger and being flexible, maintained in rest position substancially straight and in an approximatively co-axial position with respect to the tube (1) due to the resillient action of the spring (2); a flexible traction member (5) extending from the end piece (4) of the head of the catheter to the rear of the tube (1) passing through the said latter, this flexible traction member (5) being excentered with respect to the axis of this spring (2), the other end of said flexible member (5) being fixed to a control device (7).

2. Remote controlled catheter according to claim 1, caracterised by the fact that it comprises a frame member (6) having two lateral channels (11,12) permitting, the one (12) the communication with the space located between the outside of the tube (1) and the inside of the tubular covering (9), this tubular covering (9) being tightly mounted through the other of its end to the frame member (6) this tubular covering (9) being laterally perforated (13,14) to enable to liquid injected through said channel (12) to come out of the catheter; the other channel (11) permitting through the inside of the tube (1), the inflation of the baloon.

3. Remote controlled catheter according to claim 1 or claim 2, caracterised by the fact that the covering (9) and the envelope (19) are made in one piece, the common linkage separating the tubular covering (9) from the envelope (10) being tightly fixed on the tube (1).

4. Remote controlled catheter according to claim 1,2 or 3, caracterised by the fact the the tube (1) is thin and flexible.

5. Remote controlled catheter according to claim 1 caracterised by the fact that the end of the tube (1) presents the shape of a stud the outside diameter of which is less than the outside diameter of the tube (1), the coil spring (2) being fitted through its base onto the said stud shaped end ; and by the fact that the upper portion of the coil spring (2) is fitted onto the rod of the piece (4) to which one end of the flexible traction member (5) is fixed.

6. Remote controlled catheter according to claim 5 caracterised by the fact that one of the ends of the flexible traction member (5) is fixed to the head of the catheter, being clamped between the coil spring (2) and the piece (4).

**Ansprüche**

1. Ferngesteuerter Katheter mit einem Mikroballon zur Behandlung der Stenose umfassend ein Rohr (1), dessen eines Ende am unteren Teil des Katheterkopfes befestigt ist, wobei eine röhrenförmige Hülse (9) das Rohr (1) umgibt, deren eines Ende dicht am Ende des Rohres (1) befestigt ist, das den Kopf trägt, wobei diese Hülse mit der Außenwand des Rohres (1) eine Leitung zum Durchlaufen eines Fluids darstellt und dieser Kopf aus einer Schraubenfeder (2), die aus einer oder mehreren getrennten Wicklungen gebildet ist, und einem Endstück (4) zusammengesetzt ist, das am oberen Ende der Feder (2) befestigt ist und von einer Hülle (10) umgeben ist, die dicht am Ende des den Kopf tragenden Rohres (1) befestigt ist und den aufblasbaren Ballon darstellt, wobei dieser Kopf die Form eines flexiblen Hohlfingers aufweist, der im Ruhezustand durch die elastische Wirkung der Feder (2) praktisch gerade und in einer annähernd koaxial zum Rohr (1) verlaufenden Stellung gehalten wird; sich ein flexibles Zugorgan (5) vom Endstück (4) des Katheterkopfes zum hinteren Teil des Rohres (1) erstreckt und das Innere desselben durchsetzt und dieses flexible Zugorgan (5) in bezug auf die Achse dieser Schraubenfeder (2) exzentrisch ist und das andere Ende dieses flexiblen Organs (5) an einer Steuervorrichtung (7) befestigt ist.

2. Ferngesteuerter Katheter nach Anspruch 1, dadurch gekennzeichnet, daß er ein Bauelement (6) aufweist, das zwei seitliche Kanäle (11, 12) umfaßt, von denen einer (12) die Kommunikation mit dem Raum ermöglicht, der zwischen der Außenseite des Rohres (1) und dem Inneren der röhrenförmigen Hülse (9) gelegen ist, wobei diese röhrenförmige Hülse (9) mit ihrem anderen Ende dicht an dem Bauelement (6) befestigt ist, diese röhrenförmige Hülse (9) seitlich durchbohrt (13,14) ist, um zuzulassen, daß die durch diesen Kanal (12) eingespritzte Flüssigkeit an der Außenseite des Katheters austritt; wobei der andere Kanal (11) durch das Innere des Rohres (1) das Aufblasen des Ballons zuläßt.

3. Ferngesteuerter Katheter nach Anspruch 1 oder 2, dadurch gekennzeichnet, daß die Hülse (9) und die Hülle (10) einstückig ausgebildet sind und daß der gemeinsame Bereich, der die röhrenförmige Hülse (9) der Hülle (10) begrenzt, auf dem Rohr (1) dicht befestigt ist.

4. Ferngesteuerter Katheter nach Anspruch 1, 2 oder 3, dadurch gekennzeichnet, daß das Rohr (1) dünn und flexibel ist.

5. Ferngesteuerter Katheter nach Anspruch 1, dadurch gekennzeichnet, daß das Ende des Rohres (1) als Nippel bzw. Warze ausgebildet ist, dessen Außendurchmesser geringer als der Außendurchmesser des Rohres (1) ist, wobei die Schraubenfeder (2) mit ihrer Basis auf dieses warzen- bzw. nippelförmige Ende aufgestülpt bzw. aufgepreßt ist und daß der obere Teil der Schraubenfeder (2) auf den Schaft des Teiles (4) gestülpt bzw. gepreßt ist, an dem eines der Enden des flexiblen Zugorgans (5) befestigt ist.

6. Ferngesteuerter Katheter nach Anspruch 5, dadurch gekennzeichnet, daß eines der Enden des flexiblen Zugorgans (5) am Kopf des Katheters mittels Einklemmen zwischen der Schraubenfeder (2) und dem Teil (4) befestigt ist.

FIG. 1

FIG. 2

FIG. 3

EP 0 223 176 B1